# FASCICULE DE BREVET EUROPEEN

(11) **EP 0 385 826 B1**
(45) Date de publication et mention de la délivrance du brevet: **15.09.1993**
(21) Numéro de dépôt: 90400426.4
(22) Date de dépôt: 15.02.1990
(51) Int. Cl.: C07C 53/124, C07C 51/48, C12P 7/52

(54) **Procédé d'extraction de l'acide butyrique normal obtenu par fermentation**
Verfahren zur Gewinnung von durch Gärung erhaltene Normal-Buttersäure
Method of extracting normal butyric acid obtained by fermentation

(30) Priorité: 21.02.1989 FR 8902327
(43) Date de publication de la demande: 05.09.1990
(73) Titulaire: INSTITUT FRANCAIS DU PETROLE, 92502 Rueil-Malmaison (FR)
(72) Inventeur: Fayolle, Françoise, F-92240 Malakoff (FR); Marchal, Rémy, F-78400 Chatou (FR); Monot, Frédéric, F-92500 Rueil Malmaison (FR); Ballerini, Daniel, F-78100 St Germain en Laye (FR)

(56) Documents cités:
- DE-C- 804 566
- DE-C- 844 894
- US-A- 4 628 116
- CHEMICAL ABSTRACTS, tome 109, no. 21, 21 novembre 1988, abrégé no. 188678y, Columbus, Ohio, US; W. P. MURRAY et al, "Development of biotechnological processes of natural flavors and fragrances"

## Description

La présente invention concerne un procédé de séparation et de récupération d'acide butyrique normal sous forme libre ou estérifiée à partir d'un mélange contenant de l'acide n-butyrique et de l'acide acétique et plus spécialement à partir d'un moût de fermentation en solution aqueuse.

Elle s'applique particulièrement à la synthèse d'acide n-butyrique qui est utilisé comme élément de base pour les arômes dans l'industrie alimentaire et pour les parfums.

Il est connu selon le brevet US 1835700 d'isoler l'acide n-butyrique produit par voie fermentaire. La méthodologie à laquelle on a recours le plus fréquemment consiste à neutraliser le moût obtenu après fermentation, à concentrer les sels obtenus jusqu'à l'obtention d'un résidu sec et à acidifier par un acide minéral fort, le résidu afin de libérer sous forme protonée les acides organiques d'origine fermentaire. Ces derniers sont distillés et les acides n-butyrique et acétique sont répartis suivant des proportions variables dans plusieurs coupes de distillation. Elles sont ensuite refractionnées et les distillats respectifs sont combinés afin de recueillir séparément l'acide n-butyrique et l'acide acétique.

Il est encore possible d'améliorer ce procédé en diminuant la consommation de réactifs avec l'utilisation par exemple de CO2 sous pression dans l'étape de protonation du butyrate (US 1946419). Il reste malgré tout que l'ensemble de l'opération reste extrêmement coûteuse en énergie du fait de l'évaporation à sec du moût de fermentation et des nombreuses distillations en aval. La présente invention permet notamment d'éviter tout ou partie de cette évaporation.

Le brevet US - A - 4628116 décrit un procédé d'extraction d'acide butyrique et d'éthanol contenus dans un moût de fermentation par un solvant halogéné, le bromure de vinyle après une étape d'acidification à pH compris entre 3,5 et 4. Le solvant utilisé est suspecté comme étant cancérigène et il est peu recommandable pour son utilisation à une fin alimentaire.

Le brevet DE - C - 804566 décrit par ailleurs un procédé d'extraction d'acide butyrique d'origine chimique à partir d'un mélange résultant d'un traitement par l'oxygène de butyraldéhyde, l'extraction pouvant être faite notamment par des composés hydrocarbonés chlorés aromatiques ou par le cyclohexane. Le rapport solvant solution préconisé ne permet cependant de récupérer de l'acide butyrique que selon un rendement médiocre.

Enfin, l'art antérieur est illustré par le brevet DE - C - 844894 et par la publication Chemical Abstracts Vol. 109, n° 21, 21Nov 1988, page 566.

On a découvert de façon surprenante par un procédé simple et économique que l'acide n-butyrique pouvait être obtenu par extraction avec un solvant utilisé à usage alimentaire de façon très sélective avec un bon rendement et avec une pureté excellente et suffisante pour que l'acide soit directement utilisable dans l'industrie aussi bien de l'alimentation que des parfums.

L'invention concerne donc un procédé de séparation et de récupération d'acide butyrique normal à partir d'un moût de fermentation le contenant ou contenant un mélange d'acide n-butyrique et d'acide acétique ou de leurs sels, caractérisé en ce qu'il comprend les étapes successives suivantes:
a/ on acidifie le moût à pH au plus égal à 3 dans des conditions telles que l'on produit un mélange d'acides en solution aqueuse comprenant de l'acide n-butyrique et de l'acide acétique,
b/ on effectue une extraction de l'acide n-butyrique par au moins un solvant hydrocarboné aliphatique ou alicyclique ayant au moins 5 atomes de carbone et un point d'ébullition inférieur à celui de l'acide n-butyrique, ladite extraction étant effectuée dans un rapport volumique entre le solvant et la solution respectivement de 0,5:1 à 10:1 et l'on produit deux phases, une phase organique contenant la majeure partie de l'acide n-butyrique et une mineure partie d'acide acétique et une phase aqueuse, que l'on sépare ; et
c/ on évapore la phase organique et on recueille l'acide n-butyrique.

Les solvants hydrocarbonés aliphatiques ou alicycliques préconisés ont une toxicité sensiblement nulle pour l'homme et son environnement en ce sens qu'ils peuvent être utilisés à une fin alimentaire. Ce sont notamment ceux dont l'utilisation est contrôlée par la directive n° L157/28 du Journal Officiel des Communautés Européennes (24/6/88).

La solubilité du solvant dans l'eau est avantageusement inférieure à 0,5 g/l et l'eau a par ailleurs dans le solvant considéré une solubilité généralement inférieure à 2 g/l et de préférence inférieure à 0,5 g/l. Par ailleurs, le point d'ébullition du solvant choisi est avantageusement inférieur de 10°C par exemple à celui de l'acide n-butyrique (PE = 163,5°C).

On peut utiliser une coupe pétrolière d'hydrocarbures saturés ayant les trois caractéristiques ci-dessus.

On utilise avantageusement le n-pentane, le n-hexane, le n-heptane, le n-octane, le n-nonane, l'isoctane et le cyclohexane seuls ou en mélange.

De préférence l'hexane, l'heptane et le cyclohexane peuvent être utilisés en raison de leur facilité de mise en oeuvre lors des étapes d'extraction et d'évaporation.

La présente invention présente l'avantage par rapport à l'art antérieur d'éviter d'évaporer la phase aqueuse jusqu'à l'obtention d'un résidu sec et d'être un procédé économique dans la mesure où l'on évite les étapes de distillation de plusieurs fractions. Elle présente aussi l'avantage de permettre en une seule étape une extraction sélective de l'acide n-butyrique à partir d'un moût de fermentation contenant également de l'acide acétique.

On a observé de manière inattendue que le procédé selon l'invention ne s'appliquait qu'à la séparation et à la récupération d'acide n-butyrique. On a en effet remarqué que la séparation et la récupération d'acide propionique qui serait contenu dans le moût d'une fermentation propionique conduisant à un mélange d'acides propionique et acétique ne pourraient pas être effectuées selon le procédé de l'invention. Par ailleurs, la pureté de l'acide n-butyrique récupéré est excellente par rapport à la pureté du même acide récupéré par des solvants n'entrant pas dans le cadre de l'invention.

Le milieu de fermentation est généralement le suivant :

Il comprend un substrat sucré contenant des hydrates de carbone sous forme monosaccharides tels que le glucose et le fructose, provenant de l'hydrolyse acide ou enzymatique, dans des conditions connues de l'Homme de métier, les disaccharides et/ou polysaccharides contenus dans les matières premières telles que les jus sucrés obtenus par pressage ou diffusion de betteraves, de la canne à sucre et/ou du topinambour, les sirops, les égouts et les mélasses de sucrerie, les farines de céréales (blé, orge), les farines de maïs et la fécule de pomme de terre. Il est soumis à la fermentation par une souche de Clostridium choisie parmi Clostridium butyricum, Clostridium pasteurianum, Clostridium acétobutylicum, Clostridium beijerinckii et préférentiellement une souche de Clostridium tyrobutyricum telle que Clostridium tyrobutyricum IFP 923 ou CNRZ 596.

La fermentation est en général conduite à un pH compris entre 5,5 et 8,0 et à une température entre 20 et 40°C. Elle aboutit à un moût de fermentation contenant de l'acide n-butyrique et de l'acide acétique.

Le procédé est généralement applicable pour des teneurs en acide n-butyrique supérieures par exemple à 10 g/l. Pour des teneurs inférieures ou pour réaliser l'opération d'extraction dans des conditions de coût économique et de mise en oeuvre facile, on peut, selon une autre caractéristique du procédé, concentrer le moût de fermentation d'un facteur 1 à 4 et avantageusement d'un facteur 2 à 3 par exemple par évaporation du moût de fermentation ou de préférence par électrodialyse. Le dialysat obtenu est ensuite acidifié selon l'étape a/ tandis que le résidu de l'électrodialyse contenant les éléments nutritifs résiduels peut être recyclé pour la préparation du milieu d'une fermentation consécutive.

L'acidification selon l'étape a/ est généralement effectuée en présence d'un acide minéral fort tel que H₂SO₄, HCl ou H₃PO₄ à une concentration allant de l'acide pur à une solution normale. De préférence la valeur du pH est comprise entre 1,1 et 2 et plus particulièrement sensiblement égale à 1,5.

La phase d'extraction peut être effectuée en discontinu sous agitation ou avantageusement en continu et à une température comprise entre l'ambiante et la température d'ébullition du solvant choisi pour l'extraction. Le rapport en volume entre le solvant et l'eau est avantageusement compris entre 1:1 et 6:1 et permet dans ces conditions d'obtenir un bon rendement en récupération d'acide n-butyrique, avec une excellente pureté.

L'opération d'extraction en continu s'effectue habituellement à reflux de solvant dans un perforateur de Jalade avec les mêmes rapports solvant:moût de fermentation.

La phase organique contenant l'acide n-butyrique et la phase aqueuse contenant l'acide acétique sont séparés après décantation.

On peut évaporer le solvant organique selon l'étape c/ et recueillir l'acide n-butyrique, ou bien si le produit final recherché est un ester de l'acide n-butyrique, procéder à une estérification enzymatique directement dans la phase organique par action d'une lipase sans évaporer le solvant puisque les hydrocarbures saturés et en particulier l'hexane sont compatibles avec l'estérification par voie enzymatique. On procède généralement en présence d'un alcool qui est de préférence primaire ou secondaire et de façon avantageuse primaire et dont les esters de l'acide n-butyrique entrent dans la composition des arômes des parfums. Ceci inclut, par exemple, l'éthanol, l'alcool amylique, l'alcool isoamylique, le géraniol, etc...

On effectue l'estérification dans des conditions de concentration en acide n-butyrique dans le solvant d'extraction pouvant aller jusqu'à la saturation et même au-delà, la teneur en eau du solvant ne dépassant pas 0,2 %. Le rapport des concentrations molaires entre l'alcool choisi et l'acide n-butyrique est compris généralement entre 10 et 0,1, de façon avantageuse entre 2 et 0,5 et de façon préférée dans un rapport entre 1,2 et 0,9. On utilise une lipase sous forme de poudre provenant de microorganismes appartenant au genre Mucor, Candida, Aspergillus, Chromobacterium, Geotrichum, Rhizopus, Penicillium, Humicola, Alcalignes, Pseudomonas, ou du pancréas de porc, aux concentrations comprises entre 0,5 et 100 g.l⁻¹ et avantageusement entre 2,5 et 25 g.l⁻¹. La lipase peut être utilisée sous forme libre ou immobilisée suivant les méthodes connues de l'Homme de métier. La température de réaction est choisie en fonction de l'origine de la lipase utilisée.

En fin de réaction, le solvant peut être évaporé et l'ester d'acide n-butyrique récupéré.

Le procédé selon l'invention permet d'obtenir un rendement de récupération en acide n-butyrique, en général au moins égal à 40 %, avantageusement au moins égal à 70 % et de façon préférée au moins égal à 85 % avec une pureté supérieure à 97 %, par exemple entre 98 et 99 %.

L'invention sera mieux comprise à partir des exemples qui servent à illustrer l'invention sans en limiter sa portée.

### EXEMPLE 1

Les acides n-butyrique et acétique sont produits au cours d'une fermentation mettant en oeuvre la souche de Clostridium tyrobutyricum IFP 923. La fermentation est réalisée sur un milieu à base d'hydrolysat de farine de blé préparé selon le protocole suivant : un lait de farine à 38,3 % de matière sèche est préparé dans l'eau. Après avoir amené le pH à 6,2 avec NH₄OH 2N, il est soumis à une liquéfaction par réaction d'une enzyme α-amylase Termamyl 60 (NOVO) à 0,5 g/kg de farine brute pendant 2 heures à 90°C. L'étape suivante est la saccharification réalisée à pH 4,6 (pH ramené avec H₃PO₄) par action d'une enzyme amyloglucosidase AMG 150L (NOVO) à 1,6 ml/kg de farine brute pendant 66 heures à 60°C.

L'hydrolysat de farine est alors centrifugé afin d'éliminer le gluten. On obtient une teneur en glucose d'environ 370 à 380 g/l.

Le milieu de fermentation est le suivant :
· Glucose amené par l'hydrolysat de farine 2 g/l
· Extrait de levure 8,7 g/l
· (NH₄)₂SO₄ 1,7 g/l
· KH₂PO₄ 2,6 g/l
· MgSO₄, 7 H₂O 1 g/l
· FeSO₄, 7 H₂O 52 mg/l

2,3 litres de ce milieu sont placés dans un fermenteur de laboratoire de 6 litres et l'ensemble est stérilisé pendant 40 minutes à 110°C dans un autoclave, puis après refroidissement à 35°C, le pH est ajusté à 6,5 par addition d'ammoniaque à 10 % (poids/volume). Ce milieu est alors ensemencé anaérobiquement avec une préculture de la souche IFP 923.

Lorsque la concentration en glucose dans le milieu réactionnel atteint zéro, on démarre l'alimentation en hydrolysat de farine à 370 g/l de glucose à un débit horaire constant correspondant à 8 g de glucose/l de milieu réactionnel initial.

Le pH de la fermentation est maintenu à 6,5 par apport constant d'ammoniaque à 10 %.

On obtient en fin de fermentation un moût ayant une teneur en acide n-butyrique de 49,5 g.l⁻¹ et en acide acétique de 3,5 g.l⁻¹ , ce qui correspond à la transformation de 108 g.l⁻¹ de substrat glucosé.

160 ml de ce moût sont ensuite acidifiés par addition de 10 ml d'acide chlorhydrique (12 M) jusqu'à pH égal à 1,5 et soumis, sous agitation violente, à une extraction à l'hexane mettant en oeuvre un volume égal de phase organique (soit 170 ml) et de phase aqueuse.

Après séparation des phases par décantation, on récupère 23,5 g.l⁻¹ d'acide n-butyrique dans la phase hexane (soit un rendement d'extraction de 47,5 %) et 0,25 g.l⁻¹ d'acide acétique seulement dans cette même phase hexane (soit un rendement d'extraction de 7,0 %). On peut alors soit procéder à la récupération de l'acide n-butyrique à une pureté de 98,9 % par évaporation de l'hexane ou bien, si le produit final recherché est un ester, procéder à une estérification enzymatique de l'acide n-butyrique directement dans la phase organique par action d'une lipase. Pour cela, on dilue la solution d'acide n-butyrique obtenue dans l'hexane à 23,5 g.l⁻¹ comme préparée ci-dessus jusqu'à une concentration de 0,103 M. On ajoute de l'alcool amylique normal à la concentration de 0,12 M. On incorpore ensuite 42,6 mg de lipase de Mucor miehei (GIST BROCARDS) pour un volume final de 10 ml. Une solution témoin sans addition d'enzyme est réalisée parallèlement. Les deux solutions sont mises sous agitation à température de 30°C.

On peut doser la baisse de l'acidité dans l'échantillon considéré par titration comme connu de l'Homme du métier ou doser l'apparition d'ester ou la disparition de l'acide n-butyrique par chromatographie en phase gazeuse.

Après un temps de réaction de 24 heures on a formé 0,09 M de n-amylbutyrate (soit un taux de conversion de 90 %) et après un temps de réaction de 70 h on a formé 0,097 M de n-amylbutyrate (soit un taux de conversion de 97 %).

Des résultats similaires peuvent être obtenus en utilisant l'alcool isoamylique.

Après formation de l'ester, on peut récupérer celui-ci par évaporation de l'hexane.

### EXEMPLE 2

On répète l'exemple 1 en utilisant 5 volumes d'hexane par volume de moût de fermentation acidifié au lieu de 1. On récupère de cette façon dans la phase organique l'acide n-butyrique à la concentration de 7,0 g.l⁻¹compte tenu de la dilution amenée par le rapport d'extraction avec une pureté de 99 %. Les rendements d'extraction pour l'acide n-butyrique et l'acide acétique sont respectivement de 71 % et 8,5 %.

### EXEMPLE 3

Après fermentation, le moût est chauffé à la température de 100°C pendant 20 minutes. Il est ensuite centrifugé à 27 000 g pendant 20 minutes. Le moût chauffé et centrifugé est alors concentré par trois passages successifs dans un électrodialyseur jusqu'à l'obtention d'une solution concentrée à 124 g.l⁻¹ d'acide n-butyrique et 9,0 g.l⁻¹ d'acide acétique, soit un facteur de concentration de 2,5. Le moût épuisé en acides est recyclé pour la préparation d'une nouvelle charge de milieu de culture qui est fermenté sans modification de la productivité en acide n-butyrique. Le concentrat est traité comme dans l'exemple 1 à la place du moût de fermentation (rapport solvant:phase aqueuse, 1:1). On récupère alors 93,5 g.l⁻¹ d'acide n-butyrique (soit un rendement d'extraction de 75,4 %) et 1,9 g.1⁻¹ d'acide acétique (soit un rendement d'extraction de 21 %) dans la phase organique. La pureté de l'acide n-butyrique dans l'hexane est de 98 %.

Ce concentrat peut également être alternativement traité comme dans l'exemple 2 (rapport solvant:phase aqueuse, 5:1). On récupère alors dans la phase organique 21,7 g.l⁻¹ d'acide n-butyrique ce qui correspond, compte tenu de la dilution amenée par le rapport solvant:phase aqueuse, à un rendement d'extraction de 87,5 % et 0,52 g.1⁻¹ d'acide acétique, soit un rendement de 28 %. La pureté de l'acide n-butyrique obtenu dans l'hexane est de 98 %. On peut évaporer la phase organique ou procéder à une estérification enzymatique comme indiqué dans l'exemple 1.

### EXEMPLE 4 : comparatif

On répète l'exemple 3 en remplaçant l'hexane par l'acétate d'éthyl. Après extraction par 5 volumes de phase organique par volume de phase aqueuse, on récupère dans la phase organique 23 g.l⁻¹ d'acide n-butyrique (soit un rendement d'extraction de 95,5 % compte tenu de la dilution amenée par le rapport d'extraction) et 0,9 g.l⁻¹ d'acide acétique (soit un rendement d'extraction de 50 %). Le degré de pureté de l'acide n-butyrique est de 96 % seulement.

### EXEMPLE 5 : comparatif

On réalise une fermentation mettant en oeuvre une souche de Propionibacterium pensenii IP 6435. On prépare 3,6 litres d'un milieu de fermentation contenant :
· Glucose 60 g/l
· Extrait de levure 5 g/l
· NaCl 20 mg/l
· CaCl₂ 20 mg/l
· MnSO₄, H₂O 5 mg/l
· FeSO₄, 7 H₂O 30 mg/l
· KH₂PO₄ 1,5 g/l
· MgSO₄, 7 H₂O 0,6 g/l

Ce milieu placé dans un fermenteur de laboratoire de 6 litres est stérilisé pendant 40 minutes à 110°C dans un autoclave, puis après refroidissement à 35°C le pH est ajusté à 7,0 par addition d'ammoniaque à 10 % (poids/volume). Ce milieu est alors ensemencé anaérobiquement avec 400 ml de préculture de la souche IP 6435. Le pH est régulé à 7,0 par apport constant d'ammoniaque à 10 %.

En fin de fermentation, on obtient un moût contenant 19 g.l⁻¹ d'acide propionique et 13 g.l⁻¹ d'acide acétique.

On procède à une extraction comme décrit dans l'exemple 1.

Après séparation des phases par décantation, on récupère dans la phase solvant seulement 1,4 g.1⁻¹ d'acide propionique (soit un rendement d'extraction de 6 %) et 0,9 g.1⁻¹ d'acide acétique (soit un rendement d'extraction de 7 %).

### EXEMPLE 6 : extraction en continu

On répète l'exemple 3 avec le concentrat obtenu par électrodialyse en remplaçant l'extraction discontinue à l'hexane par une extraction en continu dans un perforateur de Jalade à reflux de solvant à la température de 70°C. Après 24 heures d'extraction à l'hexane à raison de 5 volumes d'hexane par volume de phase aqueuse on récupère 97 % de l'acide n-butyrique de départ (avec une pureté supérieure à 99,5 %) et 6,7 % d'acide acétique de départ.

### EXEMPLE 7

On répète l'exemple 1 en remplaçant l'hexane par d'autres hydrocarbures listés dans le tableau 1 comme des alcanes linéaire et ramifié, comme des hydrocarbures alicycliques saturé et insaturé et on effectue l'extraction comme décrit dans cet exemple. Les quantités d'acides obtenues dans les différentes phases organiques sont indiquées dans le tableau 1.

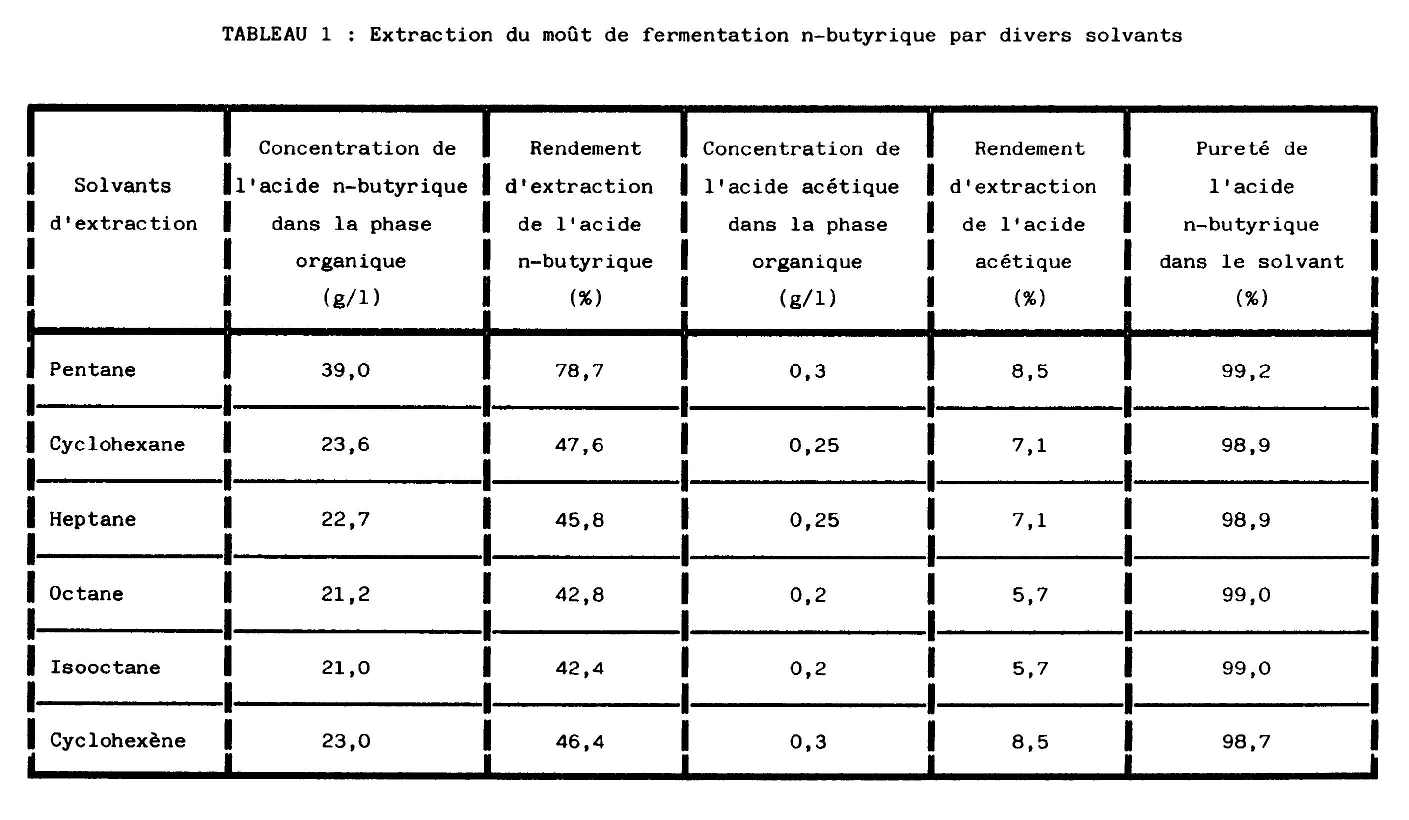

### EXEMPLES 8 à 15

On répète l'exemple 1 avec de l'hexane et on étudie l'influence du pH lors de l'étape d'acidification sur le rendement en acide butyrique obtenu. 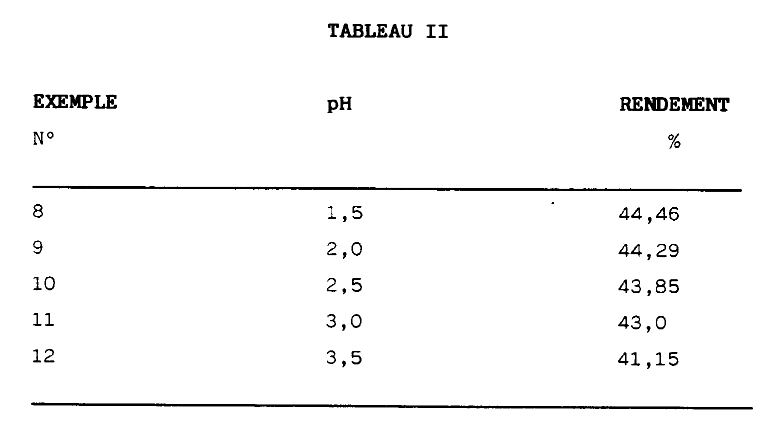
Il apparaît que les rendements obtenus sont sensiblement égaux jusqu'à pH égal à 3. Au delà de 3, les rendements commencent à chuter substantiellement.

## Revendications

1. Procédé de séparation et de récupération d'acide butyrique normal à partir d'un moût de fermentation contenant un mélange d'acide n-butyrique et d'acide acétique ou de leurs sels comprenant une étape d'acidification et une étape d'extraction par au moins un solvant approprié, caractérisé en ce que :
a/ on acidifie le moût à pH au plus égal à 3 dans des conditions telles que l'on produit un mélange d'acides en solution aqueuse comprenant de l'acide n-butyrique et de l'acide acétique,
b/ on effectue une extraction de l'acide n-butyrique par au moins un solvant hydrocarboné aliphatique ou alicyclique ayant au moins 5 atomes de carbone et un point d'ébullition inférieur à celui de l'acide n-butyrique, ladite extraction étant effectuée dans un rapport volumique entre le solvant et la solution respectivement de 0,5:1 à 10:1 et l'on produit deux phases, une phase organique contenant la majeure partie de l'acide n-butyrique et une mineure partie d'acide acétique et une phase aqueuse, que l'on sépare; et
c/ on évapore la phase organique et on recueille l'acide n-butyrique.

2. Procédé selon la revendication 1, dans lequel après l'étape b/, on effectue une estérification enzymatique directement dans la phase organique par action d'une lipase appropriée dans des conditions d'estérification, puis on évapore la phase organique selon l'étape c/ et on recueille l'acide n-butyrique sous forme d'ester.

3. Procédé selon les revendications 1 et 2 dans lequel le moût est acidifié par de l'acide chlorhydrique, de l'acide sulfurique ou de l'acide phosphorique.

4. Procédé selon l'une des revendications 1 à 3, dans lequel le rapport volumique entre le solvant et la solution aqueuse dans l'étape b/ est respectivement de 3:1 à 6:1.

5. Procédé selon l'une des revendications 1 à 4 dans lequel le solvant hydrocarboné est choisi dans le groupe formé par le n-pentane, le n-hexane, le n-heptane, le n-octane, le n-nonane, l'isooctane, le cyclohexane et leurs mélanges.

6. Procédé selon l'une des revendications 1 à 5 dans lequel, avant l'étape a/, on concentre le moût par évaporation d'un facteur 1 à 4.

7. Procédé selon l'une des revendications 1 à 5 dans lequel avant l'étape a/, on concentre le moût par électrodialyse qui produit un résidu qui est recyclé dans un milieu de culture pour une fermentation consécutive, et un dialysat qui est acidifié selon l'étape a/.

8. Procédé selon l'une des revendications 1 à 7 dans lequel l'étape d'extraction est effectuée en continu à une température comprise entre l'ambiante et celle du solvant.

9. Procédé selon l'une des revendications 1 à 8 dans lequel le moût de fermentation a une teneur en acide n-butyrique supérieure à 10g/l.

## Patentansprüche

1. Verfahren zur Abtrennung und Gewinnung von n-Butansäure ausgehend von einer Fermentationsmaische, die ein Gemisch von n-Butansäure und Essigsäure oder ihrer Salze enthält, bestehend aus einem Ansäuerungs- und einem Extraktionsschritt mit mindestens einem geeigneten Lösungsmittel, dadurch gekennzeichnet, daß:
a) die Maische auf einen pH, der höchstens gleich 3 ist, angesäuert wird, unter den Bedingungen, daß ein Gemisch der Säuren, das n-Butansäure und Essigsäure enthält, in wäßriger Lösung hergestellt wird,
b) eine Extraktion der n-Butansäure mit mindestens einem aliphatischen oder alicyclischen Kohlenwasserstoff-Lösungsmittel mit mindestens fünf Kohlenstoffatomen und einem Siedepunkt, der unterhalb dem von n-Butansäure liegt, durchgeführt wird, wobei die besagte Extraktion in einem Volumenverhältnis zwischen dem Lösungsmittel und der Lösung, respektive, von 0,5 : 1 bis 10 : 1, durchgeführt wird, und zwei Phasen hergestellt werden, eine organische Phase, die den Großteil der n-Butansäure und einen kleineren Teil der Essigsäure enthält, und eine wäßrige Phase, die abgetrennt wird; und
c) die organische Phase eingedampft und die n-Butansäure gewonnen wird.

2. Verfahren gemäß Anspruch 1, bei dem nach dem Schritt b) eine enzymatische Veresterung direkt in der organischen Phase durch Wirkung einer geeigneten Lipase unter Bedingungen zur Veresterung durchgeführt wird, dann die organische Phase gemäß Schritt c) verdampft wird und die n-Butansäure in veresterter Form gewonnen wird.

3. Verfahren gemäß den Ansprüchen 1 und 2, bei dem die Maische mit Salzsäure, Schwefelsäure oder Phosphorsäure angesäuert wird.

4. Verfahren gemäß einem der Ansprüche 1 bis 3, bei dem das Volumenverhältnis zwischen dem Lösungsmittel und der wäßrigen Lösung in Schritt b) 3 : 1 bis 6 : 1, respektive, beträgt.

5. Verfahren gemäß einem der Ansprüche 1 bis 4, bei dem das Kohlenwasserstoff-Lösungsmittel aus der Gruppe gewählt wird, die durch n-Pentan, n-Hexan, n-Heptan, n-Oktan, n-Nonan, Isooktan, Cyclohexan und ihren Mischungen gebildet wird.

6. Verfahren gemäß einem der Ansprüche 1 bis 5, bei dem vor dem Schritt a) die Maische durch Verdampfung um einen Faktor 1 bis 4 aufkonzentriert wird.

7. Verfahren gemäß einem der Ansprüche 1 bis 5, bei dem vor dem Schritt a) die Maische durch Elektrodialyse aufkonzentriert wird, die einen Rückstand erzeugt, der in einem Kulturmedium für eine anschließende Fermentation wiederverwendet wird und ein Dialysat, das gemäß Schritt a) angesäuert wird.

8. Verfahren gemäß einem der Ansprüche 1 bis 7, bei dem der Extraktionsschritt kontinuierlich bei einer Temperatur, die zwischen einschließlich Raumtemperatur und der des Lösungsmittels liegt, durchgeführt wird.

9. Verfahren gemäß einem der Ansprüche 1 bis 8, bei dem die Fermentationsmaische einen Gehalt an n-Butansäure von über 10 g/l besitzt.

## Claims

1. Process for the separation and recovery of normal butyric acid from a fermentation wort containing a mixture of n-butyric acid and acetic acid or their salts incorporating an acidification stage and an extraction stage by at least one appropriate solvent, characterized in that:
a/ the wort is acidified to a pH at the most equal to 3 under conditions such that productions takes place of a mixture of acids in aqueous solution incorporating n-butyric acid and acetic acid,
b/ an extraction takes place of the n-butyric acid by at least one aliphatic or alicytic hydrocarbon solvent having at least 5 carbon atoms and a boiling point below that of the n-butyric acid, said extraction taking place in a volume ratio between the solvent and the solution of respectively 0.5:1 to 10:1 and two phases are produced, namely an organic phase containing most of the n-butyric acid and a small amount of acetic acid, and an aqueous phase, which is separated and
c/ the organic phase is evaporated and the n-butyric acid collected.

2. Process according to claim 1, wherein following stage b/, an enzymatic esterification takes place directly in the organic phase by the action of an appropriate lipase under esterification conditions and then the organic phase is evaporated according to stage c/ and the n-butyric acid is collected in ester form.

3. Process according to claims 1 and 2, wherein the wort is acidified by hydrochloric acid, sulphuric acid or phosphoric acid.

4. Process according to any one of the claims 1 to 3, wherein the volume ratio between the solvent and the aqueous solution in stage b/ is respectively 3:1 to 6:1.

5. Process according to any one of the claims 1 to 4, wherein the hydrocarbon solvent is chosen from within the group formed by n-pentane, n-hexane, n-heptane, n-octane, n-nonane, isooctane, cyclohexane and mixtures thereof.

6. Process according to any one of the claims 1 to 5, wherein, prior to stage a/, by evaporation the wort is concentrated by a factor of 1 to 4.

7. Process according to any one of the claims 1 to 5, wherein prior to stage a/, the wort is concentrated by electrodialysis, which produces a residue, which is recycled in a culture medium for a following fermentation process, and a dialyzate, which is acidified according to stage a/.

8. Process according to any one of the claims 1 to 7, wherein the extraction stage takes place continuously at a temperature between ambient temperature and that of the solvent.

9. Process according to any one of the claims 1 to 8, wherein the fermentation wort has a n-butyric acid content above 10 g/l.
